# EUROPEAN PATENT APPLICATION

(11) **EP 3 949 966 A1**
(43) Date of publication of application: **09.02.2022**
(21) Application number: 20776399.6
(22) Date of filing: 18.03.2020
(51) Int. Cl.: A61K 31/47, A61P 35/00

(54) **CHIAURANIB FOR TREATMENT OF SMALL CELL LUNG CANCER**

(30) Priority: 25.03.2019 CN 201910229379
(71) Applicant: Shenzhen Chipscreen Biosciences Co., Ltd., Shenzhen, Guangdong 518057 (CN)
(72) Inventor: LU, Xianping, Shenzhen, Guangdong 518057 (CN); SHAN, Song, Shenzhen, Guangdong 518057 (CN); PAN, Desi, Shenzhen, Guangdong 518057 (CN); NING, Zhiqiang, Shenzhen, Guangdong 518057 (CN)
(74) Representative: Lavoix
(86) International application number: PCT/CN2020/079822
(87) International publication number: WO 2020/192506

(57) **Abstract**

Disclosed are a method for treating small cell lung cancer by using chiauranib and the use of chiauranib in the preparation of a drug for treating small cell lung cancer.

## Description

This application claims the priority of Chinese Patent Application No. 201910229379.6, filed with the China National Intellectual Property Administration on March 25, 2019, and titled with "CHIAURANIB FOR TREATMENT OF SMALL CELL LUNG CANCER", and the disclosures of which are hereby incorporated by reference in its entirety.

### FIELD

The present disclosure relates to the technical field of pharmaceuticals, specifically to use of the compound chiauranib in the treatment of small cell lung cancer.

### BACKGROUND

Both the incidence and mortality of lung cancer rank first among malignant tumors. Small cell lung cancer (SCLC) accounts for 10%~15% of the total number of lung cancers. Its clinical characteristics and biological behavior are different from other types of lung cancer, which are reflected in the short doubling time, metastasis in early stages, and extremely high degree of malignancy. Untreated patients often die within 2-4 months. Although newly-treated patients are more sensitive to chemotherapy, they are prone to drug resistance and relapse, and are relatively insensitive to second-line chemotherapy drugs, and the prognosis is poor. 30%~40% of the patients diagnosed are in the limited stage, and 60%~70% of the patients are in the extensive stage. The long-term survival rate of the limited stage is 20%, and that of the extensive stage is only 2%.

Etoposide/cisplatin regimen (EP regimen) is currently the main chemotherapy regimen for SCLC. The results of a phase III clinical study showed that in patients with limited-stage SCLC, the 2-year and 5-year survival rates of EP regimen were better than that of the cyclophosphamide/epirubicin/vincristine regimen (25% vs. 10%, 8% vs. 3%); for patients with extensive-stage SCLC, the EP regimen can also bring survival benefits. A series of subsequent studies have also confirmed the effectiveness of the EP regimen, so the EP regimen has become the standard first-line chemotherapy regimen for SCLC.

Irinotecan/cisplatin (CPT-11/DDP) (IP regimen) is another conventional chemotherapy regimen for the treatment of SCLC. In a controlled study, the IP regimen group and the EP regimen group were set up at the same time. The results showed that the objective response rates (ORR) of patients in the two groups were 84.4% and 67.5%, respectively (P=0.02), and the median survival time was 12.8 months and 9.4 months, respectively (P=0.002).

In addition to the above-mentioned conventional EP and IP regimens, there are also a few second-line treatment regimens, such as combination therapy with topotecan or paclitaxel. Studies have shown that the overall survival, quality of life and improvement of symptoms in the best supportive treatment group combined with topotecan are significantly better than those in the best supportive treatment group alone. Therefore, topotecan has become the second-line chemotherapeutic drug for SCLC.

Despite the existence of the above treatment options, in general, SCLC still lacks effective treatment methods. After the failure of conventional EP or IP regimens, there are fewer second-line options (such as topotecan, paclitaxel, etc.), and after the failure of second-line treatment, guidelines such as NCCN only recommend supportive treatment or clinical research, lacking available treatment options at this time. In addition, whether it is the EP regimen or the IP regimen, the toxicity of cisplatin often inhibits its efficacy, which may affect the treatment due to patient tolerance. Therefore, for small cell lung cancer, there is still a need to explore treatment options with better efficacy and/or fewer adverse reactions.

### SUMMARY

In view of this, the purpose of the present disclosure is to provide a treatment option with better efficacy and/or fewer adverse reactions for small cell lung cancer. The inventors unexpectedly discovered in the research that the compound of formula (I) (having a chemical name of N-(2-aminophenyl)-6-(7-methoxyquinoline-4-oxo)-1-naphthylamide, and a common name of chiauranib) can effectively treat small cell lung cancer, and has a satisfactory clinical benefit rate for small cell lung cancer, with an antitumor activity relatively significantly superior to other types of tumors such as non-small cell lung cancer and colon cancer.

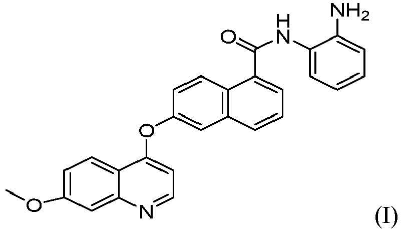

Therefore, in one aspect of the present disclosure, use of chiauranib in the manufacture of a medicament for treating small cell lung cancer is provided.

Correspondingly, the present disclosure also provides a method for treating small cell lung cancer, comprising administering chiauranib to a subject in need thereof.

In the above-mentioned pharmaceutical use and treatment method of the present disclosure, chiauranib can also be used in combination with an additional active pharmaceutical ingredient.

In some preferred embodiments of the present disclosure, the small cell lung cancer is recurrent or refractory small cell lung cancer.

The recurrent or refractory small cell lung cancer refers to the disease progression or recurrence of small cell lung cancer after receiving one or two or more different treatments, especially systemic chemotherapy regimens (such as platinum-containing chemotherapy regimens).

Chiauranib is a small molecule anti-tumor targeted drug targeting multiple protein kinases. However, there is no report on the use of chiauranib against small cell lung cancer.

We conducted a phase I clinical trial of chiauranib for patients with advanced solid tumors, enrolling a total of 18 patients with 8 types of tumor indications, including colorectal cancer, non-small cell lung cancer, gastric cancer, ovarian cancer, thyroid papillary carcinoma, diffuse large B-cell lymphoma, fibrosarcoma, and poorly differentiated renal carcinoma. The results showed that no objective remission was observed for the main clinical efficacy indicators, including cases of complete remission (CR) and partial remission (PR).

We also conducted a phase Ib clinical trial of Chiauranib for recurrent and/or refractory small cell lung cancer, enrolling 25 patients with small cell lung cancer, including 20 patients with efficacy evaluation. The results showed that the best efficacy evaluation in 4 patients was PR, and the objective response rate (ORR) was 20%. Considering the difficulty of treatment of small cell lung cancer, such treatment results are quite encouraging.

The above-mentioned experimental results show that in clinical trials of chiauranib for 8 types of tumors including non-small cell lung cancer and colon cancer, no patients with objective remission were observed for the main clinical efficacy indicators. However, chiauranib showed good efficacy in clinical trials of small cell lung cancer, with an objective response rate (ORR) of 20%, indicating that small cell lung cancer is an effective tumor indication for chiauranib. Compared with the efficacy on other types of tumors, the efficacy of chiauranib on small cell lung cancer is unexpected.

Based on the above findings, the present disclosure proposes the application of chiauranib in patients with small cell lung cancer, including but not limited to treatment of the administration of chiauranib alone, treatment of chiauranib in combination with an additional drug or adjuvant drug or therapeutic agent, as well as treatment of chiauranib in combination with other treatment(s) (such as surgery, radiotherapy, etc.).

### BRIEF DESCRIPTION OF DRAWINGS

FIG 1 shows a waterfall plot of the efficacy of chiauranib on various tumors in the phase I clinical study;
FIG 2 shows a waterfall plot of the efficacy of chiauranib on small cell lung cancer in a phase Ib clinical study.

### DETAILED DESCRIPTION

The present disclosure discloses use of chiauranib in the treatment of small cell lung cancer. Those skilled in the art can learn from the contents of the present disclosure and appropriately improve the process parameters. It should be particularly pointed out that all such alternatives and modifications are obvious to those skilled in the art and are considered to be included in the present disclosure. The applications of the present disclosure have been described by preferred examples, and apparently, those skilled in the art can make alternations or suitable modifications and combinations to the applications as described herein to achieve and apply the technology of the present disclosure without departing from the content, spirit and scope thereof.

In the present disclosure, chiauranib can be used in its prototype compound or salt form (pharmaceutically acceptable salt). The pharmaceutically acceptable salt can be prepared by using, for example, the following inorganic or organic acids: hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, hydroxymaleic acid, benzoic acid, hydroxybenzoic acid, phenylacetic acid, cinnamic acid, salicylic acid, methanesulfonic acid, benzenesulfonic acid or toluenesulfonic acid.

The pharmaceutically acceptable salt of the present disclosure can be prepared by conventional methods, for example, by dissolving the compound of the present disclosure in a water-miscible organic solvent (such as acetone, methanol, ethanol, and acetonitrile), and adding an excessive amount of aqueous solution of organic acid or inorganic acid to precipitate the salt from the resulting mixture, from which the solvent and the remaining free acid are removed, and then the precipitated salt can be separated.

Chiauranib or a pharmaceutically acceptable salt thereof of the present disclosure may include a solvate form, and preferably, the solvate is a hydrate.

In the treatment of chiauranib combined with an additional therapeutic drug, the additional therapeutic drug means that it has an effect on the treatment or prevention of cancer, including any compound or component capable of ameliorating, reducing, regulating, improving or eliminating the cause of disease, or improving symptoms, or contributing to the overall efficacy of the patient.

In the therapeutic application of the present disclosure, the administration dose of chiauranib may be 1 mg to 500 mg per day, such as 1 mg to 100 mg per day, preferably 5 mg to 80 mg per day, 5 mg to 70 mg per day, 5 mg to 60 mg per day or 5 mg to 50 mg per day, and more preferably, the daily dose may be, for example, 1 mg, 2 mg, 3 mg, 4 mg, 5 mg, 10 mg, 15 mg, 20 mg, 25 mg, 30 mg, 35 mg, 40 mg, 45 mg or 50 mg per day. The specific application dose can be adjusted according to the actual situation of the patient, the treatment regimen and the combination with other drugs.

In the therapeutic application of the present disclosure, chiauranib can be administered to a subject in a form of any suitable pharmaceutical composition. The pharmaceutical composition may be a dosage form such as oral administration or parenteral administration (including intramuscular, intravenous and subcutaneous routes), for example, capsule, tablet, granule, powder, syrup, emulsion, microemulsion, solution or suspension.

In the application of the combination therapy of the present disclosure, chiauranib and additional therapeutic agent can be administered to a subject to be treated separately, simultaneously or sequentially; chiauranib and the additional therapeutic agent can be present in the same pharmaceutical composition, or they are administered separately in the form of different pharmaceutical compositions (kits or medicine boxs). The use of chiauranib provided by the present disclosure will be further explained below.

### Example 1: Phase I clinical trial of chiauranib in the treatment of patients with advanced solid tumors

**Test drug**: chiauranib capsules, strength: 5 mg, 25 mg, produced by Shenzhen Chipscreen Biosciences, Co., Ltd.

**Dosage regimen**: The starting dose was 10 mg/day. After the starting dose, according to the modified Fibonacci method, the patients were enrolled in order from low dose to high dose, and the set dose groups were 20 mg, 35 mg, 50 mg, 65 mg...dose groups, respectively, until the MTD was determined.

Chiauranib capsules were administered orally, once a day in the morning. Before knowing the analysis data of the effects of food on bioavailability, the drugs were administered on empty stomach.

**Number of cases:** 18 cases were enrolled.

**Inclusion criteria:**
1. Patients with advanced solid tumors that had been clearly diagnosed by histology or cytology, including non-small cell lung cancer, colorectal cancer, ovarian cancer, renal cell carcinoma, gastrointestinal stromal tumor, gastric cancer, etc.;
2. Patients who had failed the standard treatment regimen or lacked the standard treatment regimen;
3. Body mass index: in the range of 18-28;
4. Age: 18-65 years old, no gender limit;
5. ECOG score: 0 or 1 point;
6. The functions of major organs met the following standards:
   a) Routine blood examination: hemoglobin (Hb) ≥100 g/L (no blood transfusion within 14 days); absolute neutrophil count (ANC) ≥1.5×10⁹/L; platelet (PLT) ≥100×10⁹/L;
   b) Biochemical examination: serum creatinine Cr ≤ upper limit of the normal value (ULN); bilirubin (BIL) ≤ 1.25×ULN; alanine aminotransferase (ALT), aspartate aminotransferase (AST) ≤ 1.5×ULN; fasting triglycerides (TG) ≤ 3.0 mmol/L, and fasting total cholesterol (TC) ≤ 7.75 mmol/L;
   c) Coagulation function: International normalized ratio (INR) <1.5.
7. For women, contraceptive measures were required during the study period and within 6 months after the end of the study, the serum or urine pregnancy test was negative within 7 days before they were enrolled, and they must be non-lactating patients; and for men, contraceptive measures were required during the study period and within 6 months after the end of the study;
8. Having voluntarily signed written informed consent form.

**Treatment plan:**
The prescribed dose of Chiauranib capsules was administered orally on empty stomach once every day, and every 28 days was a treatment cycle; there was no stopping interval during the treatment cycles, and all enrolled patients received the test drug treatment until the disease progressed or intolerable toxicity appeared.

Efficacy evaluation: The clinical benefits of tumor treatment include:
complete remission (CR)
partial remission (PR)

Judgment criteria: The efficacy was evaluated according to the RECIST version 1.1 (2009) standard of solid tumors.

Safety assessment: physical examination, vital signs, ECOG physical score, blood routine, urine routine, 12-lead ECG, blood biochemistry, electrolytes, coagulation function, myocardial enzymes, troponin, TSH, FT3, FT4, amylase, echocardiography, 24-hour urine protein quantification (if necessary), and adverse events were included.

**Clinical trial results:**
A total of 18 patients were enrolled in the trial, all of which were included in the FAS, SS, and PPS analysis sets. Among the 18 patients, 7 cases (38.9%) had colorectal cancer, 5 cases (27.8%) had non-small cell lung cancer, and 1 case each for gastric cancer, ovarian cancer, thyroid papillary carcinoma, diffuse large B-cell lymphoma, fibrosarcoma, and poorly differentiated renal carcinoma.

The results showed that no cases with objective remission were observed for the main clinical efficacy indicators, and the objective remissions included complete remission (CR) and partial remission (PR).

### Example 2: Phase Ib clinical trial of Chiauranib alone in the treatment of recurrent and refractory small cell lung cancer

**Test drug:** Chiauranib capsules, strength: 5 mg, 25 mg, produced by Shenzhen Chipscreen Biosciences, Co., Ltd.

**Dosage regimen:** Chiauranib capsules were administered at 50 mg/day, QD (no adjustments to body weight or body surface area), on an empty stomach every morning with water, swallowing whole capsules intact. Continuous administration for 28 days constituted a treatment cycle, and there was no interval between treatment cycles.

**Number of cases:** 25 cases were enrolled.

**Inclusion criteria:**
1. Age: ≥18 years old and ≤75 years old, no gender limit;
2. Patients with small cell lung cancer confirmed by histology or cytology;
3. The disease had progressed or recurred after receiving at least 2 different systemic chemotherapy regimens (including platinum-containing chemotherapy regimens) in the past;
4. According to the RECIST1.1 standard, there was at least one measurable target lesion, and the lesions treated by radiotherapy or local area treatment must have imaging evidence of disease progression before they can be regarded as target lesions;
5. ECOG physical score: 0-1 point;
6. The functions of major organs met the following standards:
   a) Blood routine: absolute neutrophil count ≥1.5×10⁹/L, platelet ≥ 75×10⁹/L, hemoglobin ≥ 80 g/L;
   b) Blood biochemistry: total bilirubin ≤ 1.5 times the upper limit of the normal value, AST/ALT ≤ 2.5 times the upper limit of the normal value (for liver metastasis, ≤ 5 times the upper limit of the normal value), serum creatinine ≤ 1.5 times the upper limit of the normal value;
   Coagulation function: Prothrombin time-international normalized ratio (PT-INR) < 1.5.
7. Expected survival time ≥ 3 months;
8. Having voluntarily signed written informed consent form.

**Treatment plan:**
The test subjects were administered with 50 mg of Chiauranib capsules orally once a day, every 28 days as a treatment cycle, and there was no stopping interval during the treatment cycles. Throughout the trial period, all subjects continued treatment until any of the following conditions (whichever occurred first) occurred: disease progression, intolerable toxic response, death, withdrawal of informed consent, or loss to follow-up.

Efficacy evaluation: According to the RECIST1.1 standard, evaluation was performed in the baseline period and at the 4th weekend after treatment, and repeated every 8 weeks until the disease progressed. Tumor imaging examinations include CT or MRI of the neck, chest, whole abdomen, and pelvis. Examinations of other parts were carried out when necessary based on clinical indications. The same techniques and methods should be employed for the baseline of the lesion and follow-up assessment and measurement.

Safety assessment: physical examination, vital signs, ECOG fitness score, blood routine, urine routine, 12-lead ECG, blood biochemistry, electrolytes, coagulation function, myocardial enzymes, troponin, TSH, FT3, FT4, amylase, echocardiography, 24-hour urine protein quantification (if necessary), and adverse events were included.

**Clinical trial results:**
25 patients with small cell lung cancer were enrolled in stages, including 20 patients with efficacy evaluation. The results showed that the best efficacy evaluation in 4 patients was PR, and the objective response rate (ORR) was 20%. These results show that Chiauranib can be effectively used in the treatment of small cell lung cancer.

### Example 3: Comparison of the efficacy of Chiauranib in the treatment of small cell lung cancer and other tumors

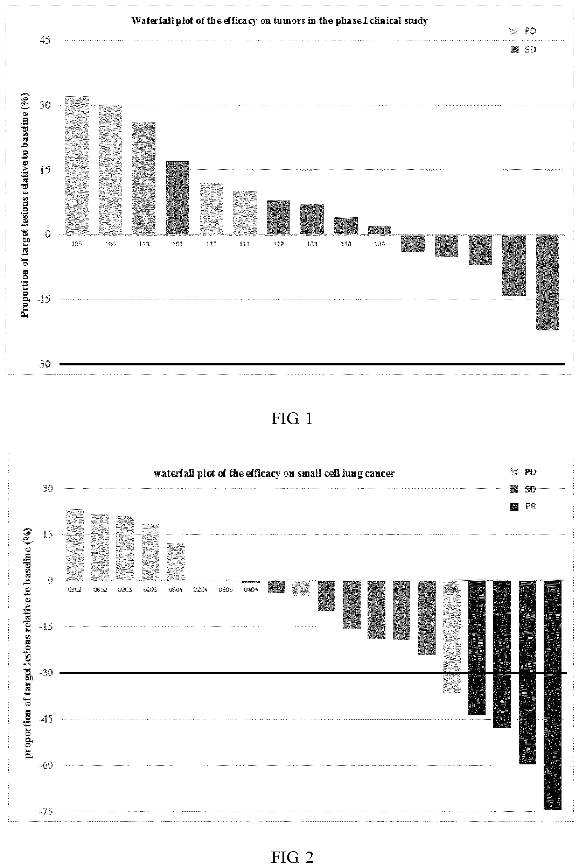

FIG 1 shows a waterfall plot of the efficacy of Chiauranib on various tumors in the phase I clinical study, including 15 patients with measurable lesions among the 18 patients enrolled. The tumor types included were colorectal cancer, non-small cell lung cancer, gastric cancer, ovarian cancer, thyroid papillary carcinoma, and poorly differentiated renal carcinoma. After treatment, there were no patients whose target lesions were reduced by more than 30% from baseline, and the target lesions were reduced by 30% from baseline is a standard for clinical objective remission of tumor treatment.

FIG 2 shows a waterfall plot of the efficacy of Chiauranib on small cell lung cancer in a phase Ib clinical study, including 20 patients with efficacy evaluation among the 25 patients enrolled. After treatment, there were 5 patients (25%) whose target lesions were reduced by more than 30% from baseline, of which 4 patients were evaluated as partial remission (PR) in clinical efficacy.

The following table shows the comparison of the efficacy of Chiauranib in the treatment of small cell lung cancer and other tumors. Chiauranib has obvious advantages in the treatment of small cell lung cancer over other tumors in terms of efficacy indicators such as objective remission, clinical benefit, and reduction of target lesions.

| Comparison items | Phase I clinical study | Phase Ib clinical study |
|---|---|---|
| Tumors types | 8 common tumors | Small cell lung cancer |
| Number of patients evaluated | 18 | 20 |
| PR (%) | 0 | **4 (20)** |
| Target lesion reduced by more than 30% | 0 | **5 (25)** |

The above description is only preferable embodiments of the present disclosure. It should be noted that those skilled in the art can make improvements and modifications without departing from the principle of the present disclosure. These improvements and modifications should also fall within the protection scope of the present disclosure.

## Claims

1. Use of chiauranib represented by the compound of formula (I) in the manufacture of a medicament for treating small cell lung cancer,

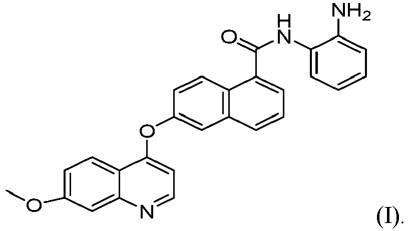

2. Use of the combination of chiauranib represented by the compound of formula (I) according to claim 1 and an additional active pharmaceutical compound in the manufacture of a medicament for treating small cell lung cancer.

3. A method for treating small cell lung cancer, comprising administering chiauranib represented by the compound of formula (I) according to claim 1 to a subject in need thereof.

4. The method according to claim 3, further comprising administering an additional therapeutic drug to the subject.
